# EUROPEAN PATENT SPECIFICATION

(11) **EP 3 207 876 B1**
(45) Date of publication and mention of the grant of the patent: **17.04.2019**
(21) Application number: 17156236.6
(22) Date of filing: 15.02.2017
(51) Int. Cl.: A61B 8/00, A61B 5/06, A61G 13/12, A61B 5/00, H01Q 1/40, A61B 6/00

(54) **BED LEVELING SYSTEMS FOR A SURGICAL TABLE**
BETTNIVELLIERUNGSSYSTEME FÜR OPERATIONSTISCH
SYSTÈMES DE MISE À NIVEAU DE LIT POUR UNE TABLE CHIRURGICALE

(30) Priority: 19.02.2016 US 201615048585
(43) Date of publication of application: 23.08.2017
(73) Proprietor: Covidien LP, Mansfield, MA 02048 (US)
(72) Inventor: GIROTTO, Darren, Louisville, CO 80027 (US); HILL, Morgan L., Boulder, CO 80301 (US)
(74) Representative: Maschio, Antonio

(56) References cited:
- US-A1- 2007 238 922
- US-A1- 2015 173 643

## Description

### BACKGROUND

### 1. Technical Field

The present disclosure relates to systems and devices for treatment of tissue. More particularly, the present disclosure relates to a bed leveling system for a surgical table, an energy emitting device, and systems for tracking locations of sensors and imaging treatment probes during the performance of a treatment procedure.

### 2. Discussion of Related Art

When treating patients, clinicians often rely on patient data including X-ray data, computed tomography (CT) scan data, magnetic resonance imaging (MRI) data, or other imaging data that allows the clinician to view the internal anatomy of a patient. The clinician utilizes the patient data to identify targets of interest and to develop strategies for accessing the targets of interest for surgical procedures.

In some instances clinicians may position a medical device between a patient's body and the surgical table to facilitate generating images of the patient's anatomy or track a precise location of a treatment probe within the patient's body and the location of the treatment probe with respect to a target for treatment. As a result, the patient may be positioned over an uneven surface, which may cause discomfort and medical complications for the patient.

Document US 2015/173643 A1 (GOVARI A., et al., 25 June 2015) may be considered to disclose a surgical table including a platform, for supporting a patient, and a bed, the bed comprising: an energy emitting device configured and dimensioned to be positioned on said platform of said surgical table; and a first spacer positioned on said platform of said surgical table to support a portion of a patient's body the first spacer having a thickness and which is illustrated in a manner that is not contradictory to it being such that a top surface of the first spacer is substantially flush with a top surface of the energy emitting device, and wherein the first spacer is adjacent the energy emitting device on the platform of the surgical table.

### SUMMARY

In this regard, it is necessary to provide a level surface for positioning a patient.

According to an example of the present disclosure, a bed leveling system for a surgical table, which incorporates a platform adapted for a patient to lie down upon, is provided. The bed leveling system includes an energy emitting device and a first spacer. The energy emitting device is configured and dimensioned to be positioned on a platform of a surgical table. The first spacer is configured to be positioned on a platform of a surgical table to support a portion of a patient's body. The first spacer has a thickness such that a top surface of the first spacer is substantially flush with a top surface of the energy emitting device when disposed adjacent the energy emitting device on a platform of a surgical table.

In some embodiments, the first spacer may include an end which is contoured corresponding to a geometry of an edge of the energy emitting device such that a substantially continuous surface is formed when the first spacer and the energy emitting device are proximately positioned.

It is contemplated that the bed leveling system may further include a second spacer configured to support a second portion of a patient's body and having a top surface that becomes substantially flush with the top surface of the energy emitting device and the top surface of the first spacer when positioned on a platform of a surgical table.

In one aspect of the present disclosure, the second spacer may include an end having a surface contour corresponding to the geometry of the energy emitting device when positioned adjacent one another.

In some embodiments, the first spacer may include a fastener configured to detachably couple to the energy emitting device.

In one aspect of the present disclosure, the fastener of the first spacer may be pivotable such that the first spacer is movably coupled to the energy emitting device.

It is envisioned that the first spacer may include a moisture barrier.

In some embodiments, the first spacer may be constructed from a rigid foam material.

It is contemplated that the first spacer may include a plurality of cells configured to form a grid structure. The grid structure may be resistant to a first force in a first direction.

In one aspect of the present disclosure, the grid structure of the first spacer may be collapsible in response to a second force in a second direction.

It is envisioned that the energy emitting device may include an EM field generator.

According to another embodiment of the present disclosure, a treatment system is provided. The treatment system includes a treatment probe, and ultrasound imager, an energy emitting device, a first spacer, a second spacer, a bed, and a tracking system. The treatment probe is configured to treat tissue and has a first tracking sensor. The ultrasound imager is configured to generate ultrasound images and has a second tracking sensor. The first spacer is configured to support a portion of a patient's body and has a thickness such that a top surface of the first spacer is substantially flush with a top surface of the energy emitting device when disposed on a platform of a surgical table. The second spacer is configured to support a second portion of a patient's body and has a top surface that becomes substantially flush with the top surface of the energy emitting device and the top surface of the first spacer when positioned on a platform of a surgical table. The bed has a bottom surface that is substantially flush with the top surfaces of each of the energy emitting device, the first spacer, and the second spacer when the energy emitting device, the first spacer, and the second spacer are disposed on a platform of a surgical table and the bed is positioned on the energy emitting device, the first spacer, and the second spacer. The tracking system is configured to receive location information from the first and second tracking sensors and to overlay the ultrasound images with a graphical representation of the treatment probe on a display based on the location information.

Any of the above aspects and embodiments of the present disclosure may be combined without departing from the scope of the present disclosure.

### BRIEF DESCRIPTION OF THE DRAWINGS

Objects and features of the presently disclosed system and method will become apparent to those of ordinary skill in the art when descriptions of various embodiments thereof are read with reference to the accompanying drawings, of which:
FIG. 1 is a schematic diagram of a tracking and treatment system in accordance with an illustrative embodiment of the present disclosure;
FIG. 2 is a side view of a portion of the surgical table of FIG. 1 including an energy emitting device and first and second spacers located thereon, in accordance with an embodiment of the present disclosure;
FIG. 3A is a view of a top surface of the energy emitting device in accordance with an embodiment of the present disclosure;
FIG. 3B is a view of a bottom surface of the energy emitting device in accordance with an embodiment of the present disclosure;
FIG. 3C is a bottom view of a sleeve for locating the energy emitting device in accordance with an embodiment of the present disclosure;
FIG. 4A is a perspective view of the first spacer of FIG. 1 in accordance with an embodiment of the present disclosure;
FIG. 4B is a bottom view of the first spacer of FIG. 1 in accordance with an embodiment of the present disclosure;
FIG. 4C is a perspective view of the second spacer of FIG. 1 in accordance with an embodiment of the present disclosure;
FIG. 4D is a bottom view of the second spacer of FIG. 1 in accordance with an embodiment of the present disclosure;
FIG. 5 is a top view of the surgical table of FIG. 1 with the energy emitting device and the first and second spacers shown in phantom located thereon in accordance with an embodiment of the present disclosure;
FIG. 6A is a side view of the first spacer of FIG. 1 in accordance with an alternative embodiment of the present disclosure;
FIG. 6B is a side view of the second spacer of FIG. 1 in accordance with an alternative embodiment of the present disclosure;
FIG. 6C is a side view of the energy emitting device of FIG. 1 in accordance with an alternative embodiment of the present disclosure;
FIG. 6D is a side view of a portion of the surgical table of FIG. 1 in accordance with an alternative embodiment of the present disclosure;
FIG. 6E is a top view of the surgical table of FIG. 1 with the energy emitting device and the first and second spacers located thereon, and with a plurality of fasteners shown in phantom, in accordance with an alternative embodiment of the present disclosure;
FIG. 7A is a top view of the surgical table of FIG. 1 with the energy emitting device and the first and second spacers located thereon, in accordance with an alternative embodiment of the present disclosure;
FIG. 7B is a detailed view of the indicated area of detail of FIG. 7A, in accordance with an alternative embodiment of the present disclosure;
FIG. 8A is a top view of the surgical table of FIG. 1 with the energy emitting device and the first and second spacers located thereon, in accordance with an alternative embodiment of the present disclosure; and
FIG. 8B is a perspective view of the first and second spacers of FIG. 8A, in accordance with an alternative embodiment of the present disclosure.

### DETAILED DESCRIPTION

While performing surgical treatment on a patient, it may be desirable to position a medical device, such as, for example, an energy emitting device and in particular an electromagnetic ("EM") field generator under the patient's body to track a precise location of a treatment probe within the patient's body, and the location of the treatment probe with respect to a target for treatment. In this regard, the present disclosure describes a system and method for leveling the surface between the surgical table and the medical device such that a bed may be positioned on a level surface.

Although the present disclosure will be described in terms of specific illustrative embodiments, it will be readily apparent to those skilled in this art that various modifications, rearrangements and substitutions may be made.

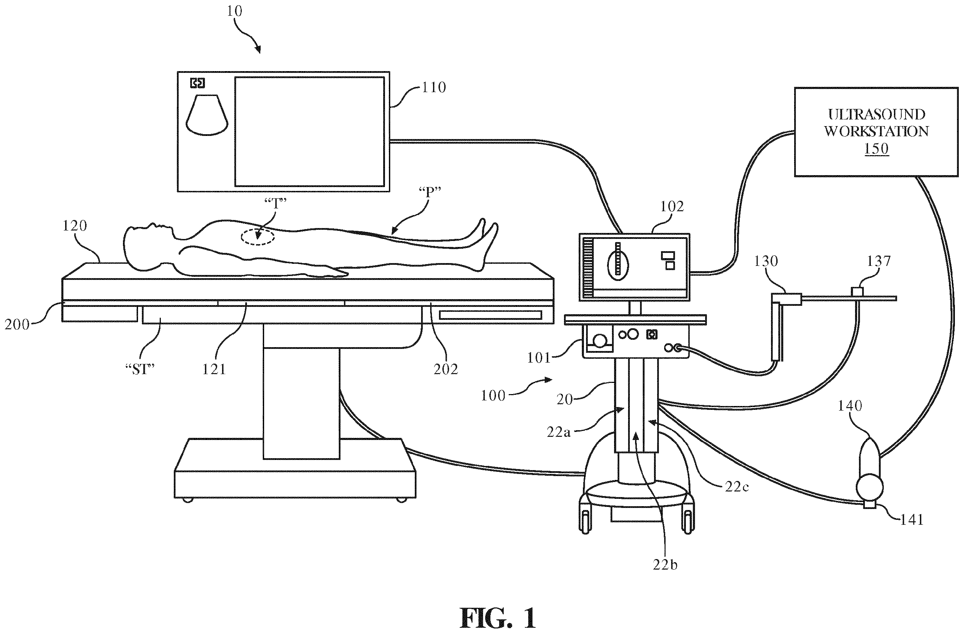

Referring now to FIG. 1, the present disclosure is generally directed to a treatment system 10 for treating a patient "P." Treatment system 10 includes a surgical cart 20 supporting an EM tracking system 100. The treatment system 10 also includes an electrosurgical generator 101, a workstation 102, a display 110, a treatment probe 130, an ultrasound imager 140, and an ultrasound workstation 150.

The electrosurgical generator 101 generates electrosurgical energy, such as, for example, radio frequency wave or microwave and provides the generated energy to the treatment probe 130. The treatment probe 130 is a surgical instrument, such as, for example, a microwave ablation antenna used to ablate and treat tissue. Various other surgical instruments or surgical tools, such as electrosurgical pencils, vessel sealers, staplers, resection devices and others, may also be used with the tracking system 100. In one embodiment, the tracking sensor 137 is located on the treatment probe 130.

The ultrasound imager 140, which may be an ultrasound wand, is used to generate ultrasound images of the patient's body during the procedure to thereby provide visualization of the location of surgical instruments, such as the treatment probe 130, inside the patient's body. In one embodiment, the tracking sensor 141 is located on the ultrasound imager 140.

The EM tracking system 100 interacts with multiple devices, such as an EM field generator 121 and the one or more tracking sensors 137, 141, such as, for example, an EM sensor, and the display 110 on which a user interface presents the location of the tracking sensors 137, 141 in an EM field in combination with one or more imaging modalities. The EM tracking system 100 includes software which converts signals received from the tracking sensors 137, 141 and performs necessary calculations to track the location of the EM sensors in an EM field generated by the EM field generator 121. In addition to tracking information, the display 110 presents to a user the results of the software processing including instructions, images, and messages relating to the performance of the procedure.

According to an embodiment, EM field generator 121 or another energy emitting device associated with treatment system 10 is disposed on or built into a platform, such as, for example, a surgical table "ST." During a treatment procedure, patient "P" is located on a bed 120 adjacent EM field generator 121. In an embodiment, EM field generator 121 is configured to generate an EM field through a portion of the anatomy of patient "P," which includes a target "T," for example, through patient "P's" torso, to enable navigation to and treatment of all the major organs of patient "P's" anatomy. It will be appreciated that in some embodiments, another location in patient "P" may serve as the area of interest.

An example of an EM field generator 121 is the AURORA™ system sold by Northern Digital Inc. However, alternative EM field generators may be implemented in other embodiments.

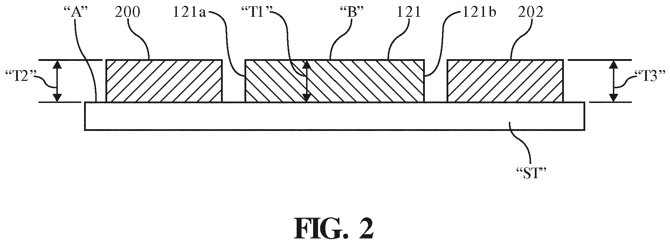

To create a leveled surface, a first spacer 200 and a second spacer 202 are located on surgical table "ST," as illustrated, for example, in FIGS. 1, 2, 5, 6E, and 7A. In an embodiment, first spacer 200 is located on top surface "A" of surgical table "ST" adjacent a first end 121a of EM field generator 121 and second spacer 202 is located on top surface "A" of surgical table "ST" adjacent an opposing second end 121b of EM field generator 121. Alternatively, the location of spacers 200, 202 may be interchangeable relative to EM field generator 121. Further, although two spacers are shown in the figures, it is contemplated that any number of spacers may be used as necessary to create a leveled surface on surgical table "ST."

Each of spacers 200, 202 has a thickness "T2," "T3," respectively, that is substantially equal to a thickness "T1" of EM field generator 121. For example, in an embodiment in which thickness "T1" of EM field generator 121 is approximately 1.34 inches (3.4036 centimeters), thicknesses "T2," "T3" of spacers 200, 202, respectively, are also approximately 1.34 inches (3.4036 centimeters). Notwithstanding the particular thicknesses of spacers 200, 202, and EM field generator 121, it is contemplated that spacers 200, 202 are flush with top surface "B" of EM field generator 121.

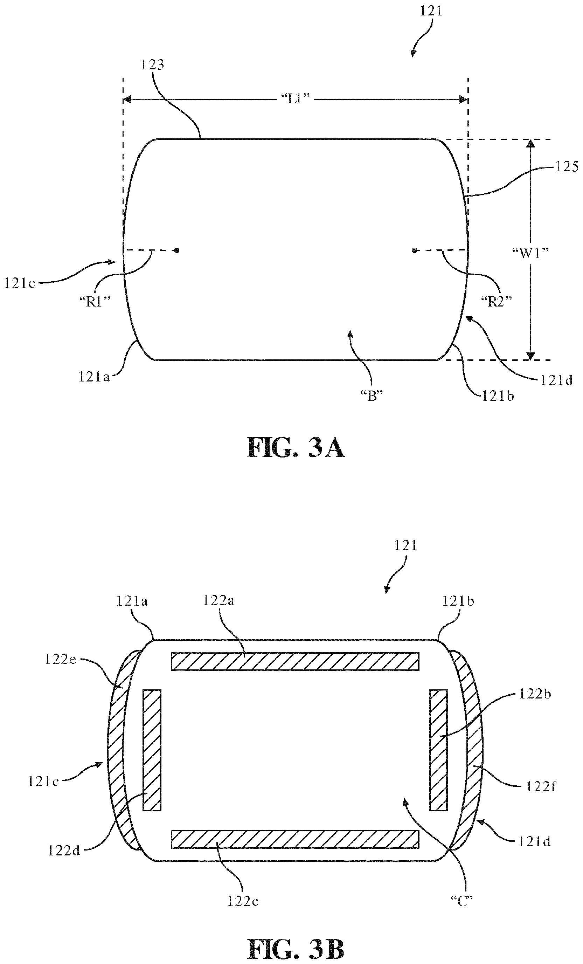

With reference to FIG. 3A, a top view of top surface "B" of EM field generator 121 is illustrated. First end 121a and second end 121b of EM field generator 121 includes a surface 121c and a surface 121d, respectively, wherein surfaces 121c, 121d are configured to be located adjacent spacers 200, 202. In an embodiment, first and second ends 121a, 121b are curved to define a first radius of curvature "R1" and a second radius of curvature "R2," respectively. In other embodiments, first and second ends 121a, 121b may include complex geometries. EM field generator 121 also includes a first side 123 and a second side 125 that defines a length "L1" and a width "W1," respectively. In an embodiment, length "L1" of EM field generator 121 is approximately 30 inches (76.2 centimeters) and width "W1" of the EM field generator 121 is approximately 20 inches (50.8 centimeters). However, it is contemplated EM field generator 121 may be any suitable size for the treatment of patient "P."

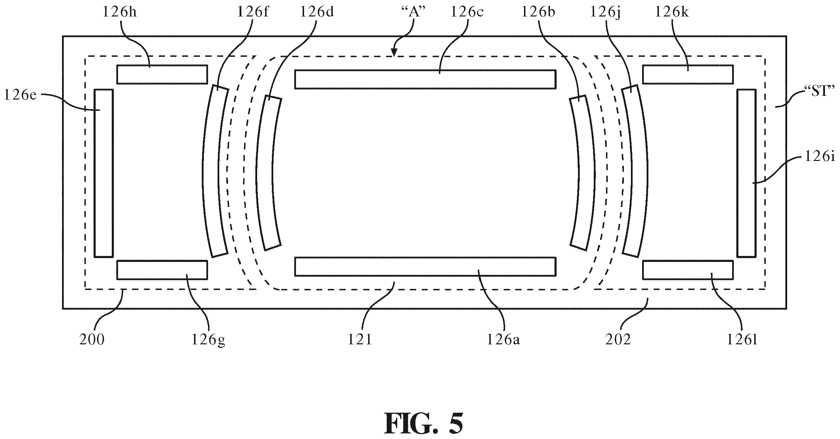

Turning now to FIG. 3B, a bottom surface "C" of EM field generator 121 is illustrated. Bottom surface "C" of EM field generator 121 includes a plurality of fasteners 122a, 122b, 122c, 122d configured to detachably couple EM field generator 121 to surgical table "ST." Specifically, as illustrated in FIG. 5, top surface "A" of surgical table "ST" includes a plurality of fasteners 126a, 126b, 126c, 126d that are configured to engage and detachably coupled with the respective plurality of fasteners 122a, 122b, 122c, 122d on bottom surface "C" of EM field generator 121. In an embodiment, as illustrated in FIG. 3B, fasteners 122a, 122b, 122c, 122d are disposed on bottom surface "C" adjacent a perimeter of EM field generator 121. Alternatively, fasteners 122a, 122b, 122c, 122d may be disposed on bottom surface "C" such that fasteners 122a, 122b, 122c, 122d are aligned in rows. Notwithstanding the positioning of fasteners 122a, 122b, 122c, 122d of EM field generator 121, fasteners 126a, 126b, 126c, 126d are correspondingly positioned on top surface "A" of surgical table "ST" such that EM field generator 121 is detachably coupled to surgical table "ST."

Although four pairs of fasteners are illustrated, it is contemplated that any suitable number of fasteners may be used. In some embodiments, the plurality of fastener pairs 122a, 126a, 122b, 126b, 122c, 126c, 122d, 126d may be constructed from hook-and-loop type fasteners, such as, for example, VELCRO® brand fasteners (a registered trademark owned by Velcro Industries B.V.), though other suitable adhesives are also contemplated.

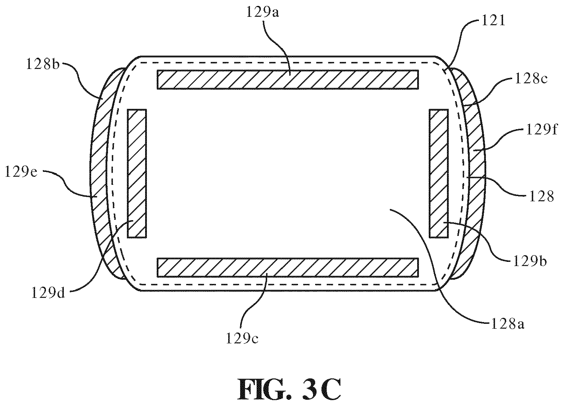

In another embodiment, as illustrated in FIG. 3C, EM field generator 121 (shown in phantom) is disposed within a sleeve 128 correspondingly sized to locate EM field generator 121 therein. It is contemplated that a bottom surface 128a of sleeve 128 includes a plurality of fasteners 129a, 129b, 129c, 129d similar to fasteners 122a, 122b, 122c, 122d detailed above. Accordingly, fasteners 129a, 129b, 129c, 129d of sleeve 128 are configured to detachably fix EM field generator 121 to surgical table "ST" when EM field generator 121 is disposed within sleeve 128.

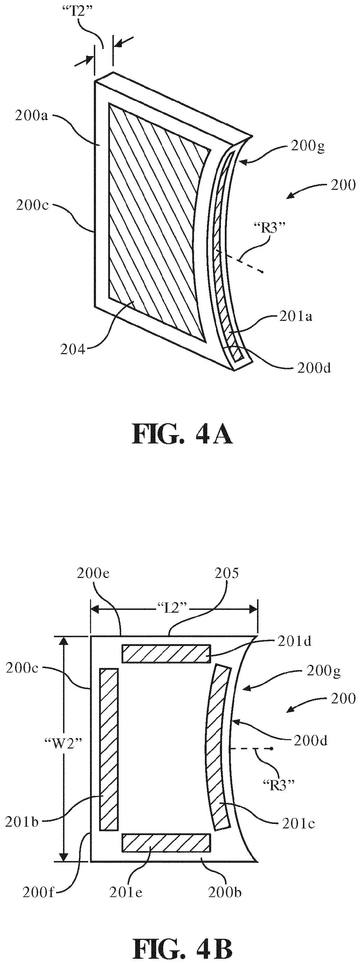

FIGS. 4A and 4B are perspective and bottom views of first spacer 200, respectively, according to an embodiment of the present disclosure. First spacer 200 has a top surface 200a and a bottom surface 200b, wherein top surface 200a is configured to contact bed 120 and bottom surface 200b is configured to contact surgical table "ST." First spacer 200 also has a first end 200c and a second end 200d, wherein second end 200d of first spacer 200 includes a radius of curvature "R3" matching the radius of curvature "R1" of first end 121a of EM field generator 121 and thereby corresponding to the geometry of first end 121a of EM field generator 121. In other embodiments, second end 200d of first spacer 200 may include complex geometry matching the complex geometry of first end 121a of EM field generator 121. First spacer 200 also includes a first side 200e and a second side 200f that defines a length "L2" and a width "W2," respectively. In an embodiment, width "W2" of first spacer 200 corresponds to width "W1" of the EM field generator 121. For example, in an embodiment where width "W1" of EM field generator 121 is 20 inches (50.8 centimeters), width "W2" of first spacer 200 may also be 20 inches (50.8 centimeters).

In an embodiment, first spacer 200 includes a plurality of fasteners 201a, 201b, 201c, 201d, 201e. For example, fastener 201a is located on a surface 200g on second end 200d of first fastener 200. In an embodiment, EM field generator 121 includes a corresponding fastener 122e located on surface 121c of first end 121a configured to engage fastener 201a of first spacer 200 such that first spacer 200 is detachably coupled to EM field generator 121. In some embodiments, sleeve 128 includes a fastener 129e (see FIG. 3C) located on a first end 128b thereof, such that when EM field generator 121 is disposed within sleeve 128, EM field generator 121 is detachably coupled to first spacer 200 via fastener 129e of sleeve 128.

Additional fasteners 201b, 201c, 201d, 201e are disposed on bottom surface 200b of first spacer 200 and are configured to detachably couple first spacer 200 to surgical table "ST." Specifically, as illustrated in FIG. 5, top surface "A" of surgical table "ST" includes a plurality of fasteners 126e, 126f, 126g, 126h that are configured to engage and detachably couple with the respective fasteners 201b, 201c, 201d, 201e on bottom surface 200b of first spacer 200. In an embodiment as illustrated in FIG. 4B, fasteners 201b, 201c, 201d, 201e are disposed on bottom surface 200b adjacent the perimeter of first spacer 200. In some embodiments, fasteners 201b, 201c, 201d, 201e may be disposed on bottom surface 200b such that fasteners 201b, 201c, 201d, 201e are aligned in rows. Alternatively, fasteners 201b, 201c, 201d, 201e may be disposed on first spacer 200 such that fasteners 201b, 201c, 201d, 201e cover the entire bottom surface 200b of first spacer 200. Notwithstanding the positioning of fasteners 201b, 201c, 201d, 201e on first spacer 200, fasteners 126e, 126f, 126g, 126h are correspondingly positioned on top surface "A" of surgical table "ST" such that first spacer 200 is detachably coupled to surgical table "ST."

Although five fasteners are illustrated on first spacer 200, it is contemplated that any suitable number of fasteners on first spacer 200 (and corresponding fasteners on surgical table "ST") may be used. In an embodiment, the plurality of fasteners 201a, 201b, 201c, 201d, 201e may be constructed from hook-and-loop type fasteners, such as, for example, VELCRO® brand fasteners (a registered trademark owned by Velcro Industries B.V.), though other suitable adhesives are also contemplated.

Returning briefly to FIG. 4A, in an embodiment, top surface 200a of first spacer 200 includes a moisture barrier 204 configured to prevent moisture from diffusing through bed 120 and into first spacer 200. According to an embodiment, moisture barrier 204 is a layer of material forming top surface 200a of first spacer 200 or is a separate piece placed over top surface 200a of first spacer 200. In another embodiment, moisture barrier 204 is a layer of material embedded in first spacer 200 to form at least a portion of top surface 200a. Though it is not explicitly illustrated, in an alternative embodiment, moisture barrier 204 may encase first spacer 200 entirely. Moisture barrier 204 may be constructed from any moisture-impermeable material such as, for example, water resistant fabrics, plastic, silicone, rubber, vinyl, and the like. Moisture barrier 204 may also be treated with silver, making it antimicrobial.

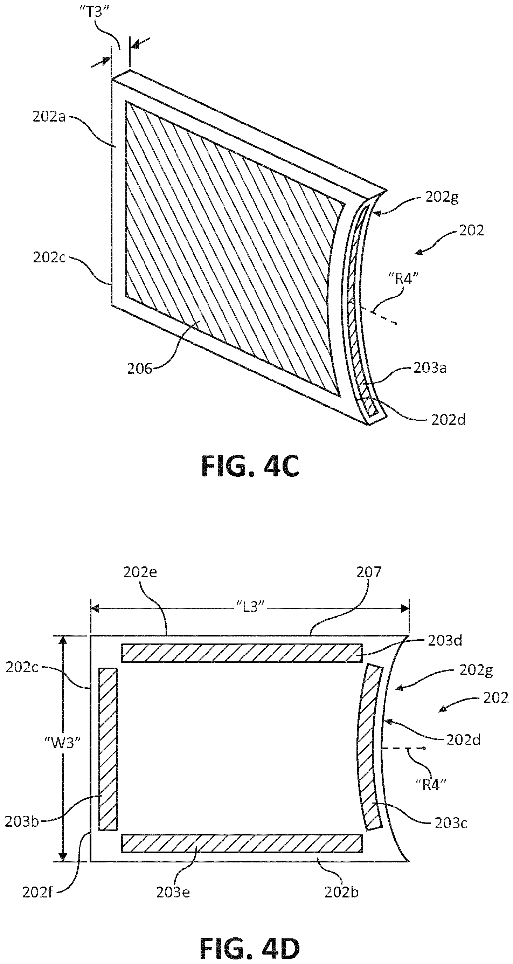

FIGS. 4C and 4D are perspective and bottom views of second spacer 202, respectively, according to an embodiment of the present disclosure. Similar to first spacer 200, second spacer 202 has a top surface 202a and a bottom surface 202b, wherein top surface 202a is configured to contact bed 120 and bottom surface 202b is configured to contact surgical table "ST."

Second spacer 202 has a first end 202c and a second end 202d, wherein second end 202d of second spacer 202 includes a radius of curvature "R4" matching the radius of curvature "R2" of second end 121b of EM field generator 121 and thereby corresponding to the geometry of second end 121b of EM field generator 121. In other embodiments, second end 202d of second spacer 202 may include complex geometry matching the complex geometry of second end 121b of EM field generator 121. Second spacer 202 includes a first side 202e and a second side 202f that defines a length "L3" and a width "W3," respectively. In an embodiment, width "W3" of second spacer 202 corresponds to width "W1" of the EM field generator 121. For example, in an embodiment where width "W1" of EM field generator 121 is 20 inches (50.8 centimeters), width "W3" of second spacer 202 may also be 20 inches (50.8 centimeters). In an embodiment, length "L3" of second spacer 202 is greater than length "L2" of first spacer 200. In another embodiment, length "L2" of first spacer 200 is greater than length "L3" of second spacer 202. Alternatively, lengths "L3" and "L4" are equal.

Second spacer 202 includes a plurality of fasteners 203a, 203b, 203c, 203d, 203e. For example, fastener 203a is located on a surface 202g on second end 202d of second spacer 202. In an embodiment, EM field generator 121 includes a corresponding fastener 122f located on surface 121d of second end 121b configured to engage fastener 203a of second spacer 202 such that second spacer 202 is detachably coupled to EM field generator 121. In some embodiments, sleeve 128 includes a fastener 129f (see FIG. 3C) located on a second end 128c thereof, such that when EM field generator 121 is disposed within sleeve 128, EM field generator 121 is detachably coupled to second spacer 202 via fastener 129f of sleeve 128.

Additional fasteners 203b, 203c, 203d, 203e are disposed on bottom surface 202b of second spacer 202 and are configured to detachably couple second spacer 202 to surgical table "ST." Specifically, as illustrated in FIG. 5, top surface "A" of surgical table "ST" includes a plurality of fasteners 126i, 126j, 126k, 1261 that are configured to engage and detachably couple with the respective fasteners 203b, 203c, 203d, 203e on bottom surface 202b of second spacer 202. In an embodiment as illustrated in FIG. 4D, fasteners 203b, 203c, 203d, 203e are disposed on bottom surface 202b adjacent a perimeter of second spacer 202. Alternatively, fasteners 203b, 203c, 203d, 203e may be disposed on bottom surface 202b of second spacer 202 in orientations similar to those detailed above with respect to fasteners 201b, 201c, 201d, 201e on bottom surface 200b of first spacer 200.

Notwithstanding the positioning of fasteners 203b, 203c, 203d, 203e on second spacer 202, fasteners 126i, 126j, 126k, 1261 are correspondingly positioned on top surface "A" of surgical table "ST" such that second spacer 202 is detachably coupled to surgical table "ST." Further, similar to first spacer 200, it is contemplated that any suitable number of fasteners on second spacer 202 (and corresponding fasteners on surgical table "ST") may be used. In an embodiment, the plurality of fasteners 203a, 203b, 203c, 203d, 203e on second spacer 202 may be constructed from hook-and-loop type fasteners, such as, for example, VELCRO® brand fasteners (a registered trademark owned by Velcro Industries B.V.), though other suitable adhesives are also contemplated.

As shown in FIG. 4C, in an embodiment, top surface 202a of second spacer 202 includes a moisture barrier 206 similar to moisture barrier 204 of first spacer 200. As such, moisture barrier 206 may be constructed from any moisture-impermeable material such as, for example, water resistant fabrics, plastic, silicone, rubber, vinyl, and the like. Moisture barrier 206 may be treated with silver, making it antimicrobial. Though it is not explicitly illustrated, in an alternative embodiment, moisture barrier 206 may encase second spacer 202 entirely.

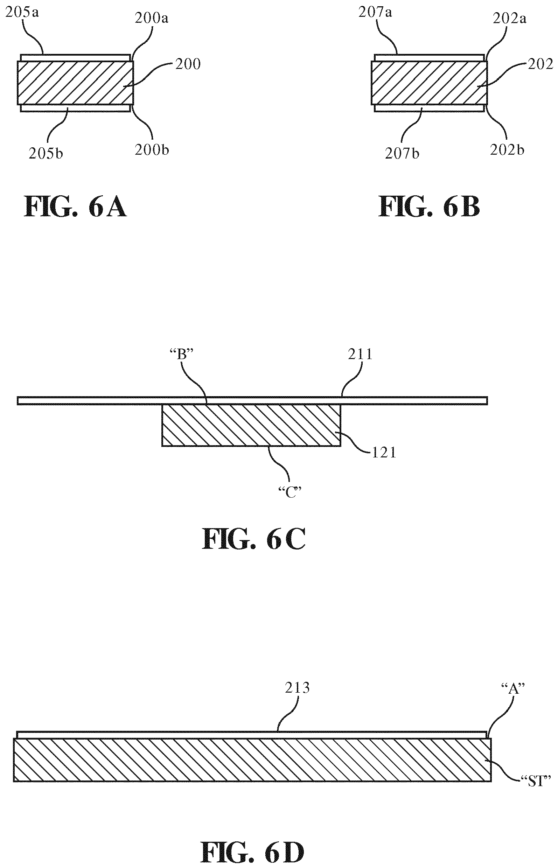

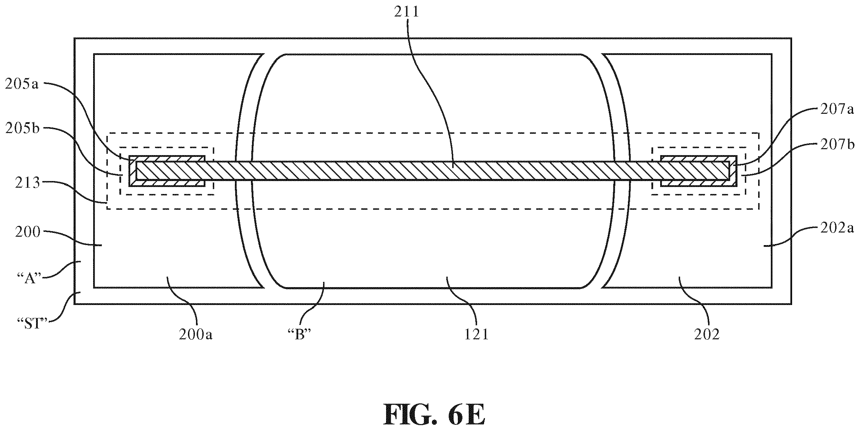

Turning now to FIGS. 6A-6E, alternative fastener arrangements for spacers 200, 202, EM field generator 121, and surgical table "ST" are illustrated. In this embodiment, first spacer 200 includes a fastener 205a and a fastener 205b positioned on top surface 200a and bottom surface 200b thereof, respectively. In some embodiments, fasteners 205a, 205b are positioned to be centered relative to width "W2" (see FIG. 4B) of first spacer 200. However, alternative positions for fasteners 205a, 205b relative to width "W2" of first spacer 200 are also contemplated. Similarly, second spacer 202 includes a fastener 207a and a fastener 207b positioned on top surface 202a and bottom surface 202b thereof, respectively. In some embodiments, fasteners 207a, 207b are positioned to be centered relative to width "W3" (see FIG. 4D) of second spacer 202. However, alternative positions for fasteners 207a, 207b relative to width "W3" of second spacer 202 are also contemplated. As shown in FIG. 6C, EM field generator 121 includes a fastener 211 positioned on top surface "B" thereof. Similar to the fasteners of spacers 200, 202, fastener 211 is positioned to be centered relative to width "W1" (see FIG. 3A) of EM field generator 121. However, alternative positions for fastener 211 relative to width "W1" of EM field generator 121 are also contemplated. As shown in the top view in FIG. 6E, fastener 211 of EM field generator 121 is configured to engage fasteners 205a, 207a on top surfaces 200a, 202b of spacers 200, 202, respectively, to detachably secure EM field generator 121 to spacers 200, 202.

In this embodiment, surgical table "ST" includes a fastener 213 positioned on top surface "A" thereof (see FIG. 6D). As shown in the top view in FIG. 6E, fastener 213 of surgical table "ST" is configured to engage fasteners 205b, 207b on bottom surfaces 200b, 202b of fasteners 200, 202, respectively, to detachably fix spacers 200, 202 to top surface "A" thereof. As such, fastener 213 is positioned on surgical table "ST" to correspond to the position of fasteners 205b, 207b on bottom surfaces 200b, 202b of fasteners 200, 202, respectively. In an embodiment, the plurality of fasteners 205a, 205b, 207a, 207b, 211, 213 on spacers 200, 202, EM field generator 121, and surgical table "ST," respectively, may be constructed from hook-and-loop type fasteners, such as, for example, VELCRO® brand fasteners (a registered trademark owned by Velcro Industries B.V.), though other suitable adhesives are also contemplated.

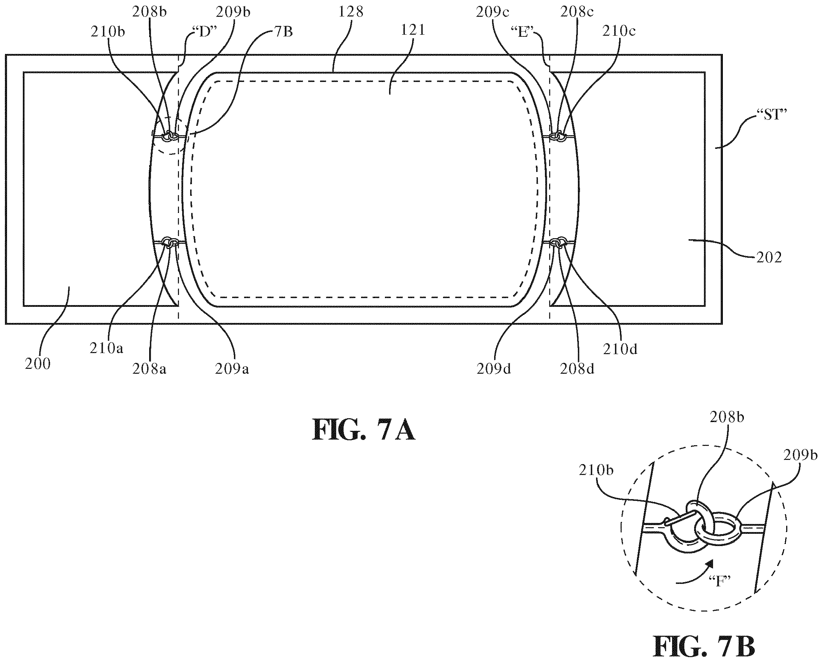

Turning now to FIGS. 7A and 7B, in some embodiments, surgical table "ST" may be a bariatric bed and include pivotable portions along dotted lines "D" and "E." In this embodiment, it may be desirable for first and second spacers 200 and 202 to remain movable, such as, for example, to be pivotable with respect to EM field generator 121 while still remaining detachably coupled to EM field generator 121 when an orientation of surgical table "ST" changes from a flat orientation shown in FIG. 7A to a bended orientation (not illustrated). To that end, first and second spacers 200 and 202 includes a plurality of hooks 208a, 208b, 208c, 208d configured to engage with a plurality of corresponding loops 209a, 209b, 209c, 209d, respectively, of sleeve 128. With specific reference to FIG. 6B, hook 208b and loop 209b are shown in detail as an example. Hook 208b includes a latch 210b resiliently biased in a direction indicated by arrow "F" to return hook 208b to a secured position shown in FIG. 6B to secure loop 209b therein. Similar to hook 208b, the remaining plurality of hooks 208a, 208c, 208d each include a latch 210a, 210c, 210d, respectively.

In some embodiments, first and second spacers 200, 202 are constructed from semi-rigid materials. Suitable materials include, but are not limited to, polystyrene made into foam, plastic, silicone, and other suitable semi-rigid materials that can be sterilized and reused.

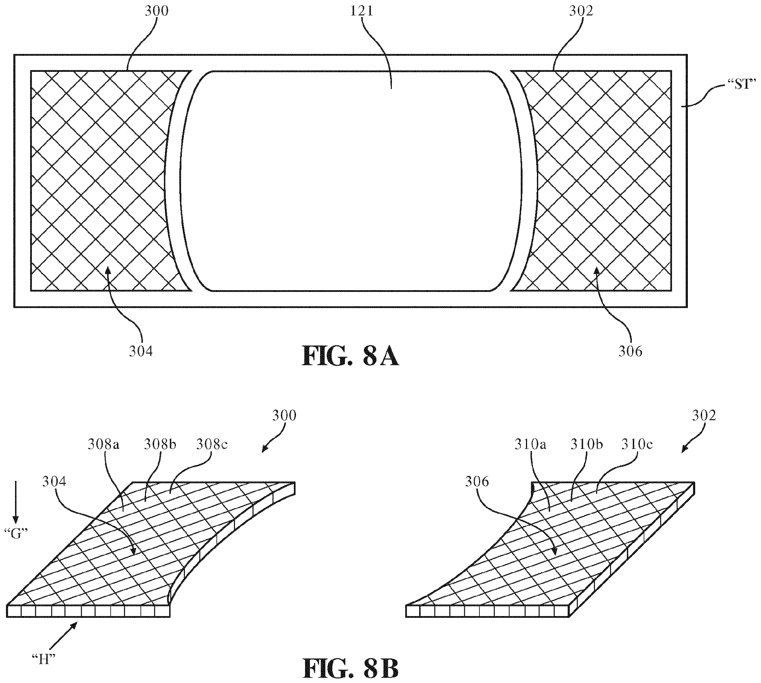

In another embodiment, as shown in FIGS. 8A and 8B, a first spacer 300 and a second spacer 302 similar to first and second spacers 200, 202 include a grid or honeycomb structure 304, 306, respectively. Specifically, first spacer 300 includes a plurality of cells 308a, 308b, 308c that are connected to thereby create grid structure 304. Similarly, second spacer 302 includes a plurality of cells 310a, 310b, 310c that are connected to thereby create grid structure 306. Though only three cells are indicated in each of spacers 300, 302, it is contemplated that spacers 300, 302 may include any number of cells necessary to create grid structures 304, 306. It is contemplated that the configuration of cells 308a/310a, 308b/310b, 308c/310c are such that grid structures 304, 306 are able to withstand force in a direction indicated by arrow "G" but are not able to withstand force in a direction indicated by arrow "H." In other words, grid structures 304, 306 are configured to provide rigid support for patient "P" where force is being applied by the anatomy of patient "P" on grid structures 304, 306 in the direction indicated by arrow "G." However, when force is applied on grid structures 304, 306 in the direction indicated by arrow "H," grid structures 304, 306 are collapsible such that spacers 300, 302 may be easily stored.

In operation, with reference to FIGS. 1-8B, surgical table "ST" is set up as shown in FIG. 1 prior to locating patient "P" thereon for treatment. In an embodiment in which EM field generator 121 is not built into surgical table "ST," EM field generator 121 is disposed on top surface "A" of surgical table "ST," and secured thereto with the plurality of fasteners 122a, 122b, 122c, 122d located on bottom surface "C" of EM field generator 121. Next, first and second spacers 200 and 202 are detachably fixed to EM field generator 121 using the plurality of fasteners 201a, 203a to engage the corresponding plurality of fasteners 122e, 122f of EM field generator 121. First and second spacers 200, 202 are then secured to top surface "A" of surgical table "ST," using fasteners 201b, 201c, 201d, 201e on bottom surface 200b of first spacer 200 and fasteners 203b, 203c, 203d, 203e on bottom surface 202b of second spacer 202 to engage respective fasteners 126e, 126f, 126g, 126h, 126i, 126j, 126k, 1261 on top surface "A" of surgical table "ST." Bed 120 is then placed over spacers 200, 202 and EM field generator 121.

In another embodiment as illustrated in FIGS. 6A-6E, EM field generator 121 is disposed on top surface "A" of surgical table "ST" and first and second spacers 200, 202 are positioned on surgical table "ST," adjacent EM field generator 121, on opposing ends thereof. First and second spacers 200, 202 are secured to top surface "A" of surgical table "ST," using fastener 205b on bottom surface 200b of first spacer 200 and fastener 207b on bottom surface 202b of second spacer 202 to engage respective fastener 213 on top surface "A" of surgical table "ST." Next, EM field generator 121 is detachably secured to first and second spacers 200, 202 using fastener 211 on top surface "B" of EM field generator 121 to engage fasteners 205a, 207a on top surfaces 200a, 202a of first and second spacers 200, 202, respectively. Bed 120 is then placed over spacers 200, 202 and EM field generator 121.

Though not specifically shown, in some embodiments, bed 120 includes mechanisms, such as, for example, straps, fasteners, and the like, for releasably attaching to spacers 200, 202 and EM field generator 121 such that bed 120 does not move during the treatment of patient "P." After completing the treatment, patient "P," is removed from bed 120. Bed 120 is then removed from spacers 200, 202 and EM field generator 121. Spacers 200, 202 are then detached from EM field generator 121 and surgical table "ST" and EM field generator 121 is removed from surgical table "ST." Finally, spacers 200, 202 and EM field generator 121 are located in compartments 22a, 22b, 22b within cart 20 for storage.

Although embodiments have been described in detail with reference to the accompanying drawings for the purpose of illustration and description, it is to be understood that the inventive processes and apparatus are not to be construed as limited thereby. The disclosure is described by the following numbered paragraphs:-
1. A bed leveling system for a surgical table, which incorporates a platform adapted for a patient to lie down upon, comprising:
   an energy emitting device configured and dimensioned to be positioned on a platform of a surgical table; and
   a first spacer configured to be positioned on a platform of a surgical table to support a portion of a patient's body, the first spacer having a thickness such that a top surface of the first spacer is substantially flush with a top surface of the energy emitting device when disposed adjacent the energy emitting device on a platform of a surgical table.
2. The bed leveling system according to paragraph 1, wherein the first spacer includes an end which is contoured corresponding to a geometry of an edge of the energy emitting device such that a substantially continuous surface is formed when the first spacer and the energy emitting device are proximately positioned.
3. The bed leveling system according to paragraph 1, further comprising a second spacer configured to support a second portion of a patient's body and having a top surface that becomes substantially flush with the top surface of the energy emitting device and the top surface of the first spacer when positioned on a platform of a surgical table.
4. The bed leveling system according to paragraph 3, wherein the second spacer has an end having a surface contour corresponding to the geometry of the energy emitting device when positioned adjacent one another.
5. The bed leveling system according to paragraph 1, wherein the energy emitting device includes a fastener configured to detachably couple to a platform of a surgical table.
6. The bed leveling system according to paragraph 1, wherein the first spacer includes a fastener configured to detachably couple to the energy emitting device.
7. The bed leveling system according to paragraph 1, wherein the first spacer includes a fastener configured to detachably couple to a platform.
8. The bed leveling system according to paragraph 6, wherein the fastener of the first spacer is pivotable such that the first spacer is movably coupled to the energy emitting device.
9. The bed leveling system according to paragraph 1, wherein the first spacer includes a moisture barrier.
10. The bed leveling system according to paragraph 1, wherein the first spacer is constructed from a rigid foam material.
11. The bed leveling system according to paragraph 1, wherein the first spacer includes a plurality of cells configured to form a grid structure, the grid structure being resistant to a first force in a first direction.
12. The bed leveling system according to paragraph 11, wherein the grid structure of the first spacer is configured to be collapsible in response to a second force in a second direction.
13. The bed leveling system according to paragraph 1, wherein the energy emitting device includes an EM field generator.
14. A treatment system comprising:
   a treatment probe configured to treat tissue and having a first tracking sensor;
   an ultrasound imager configured to generate ultrasound images and having a second tracking sensor;
   an energy emitting device;
   a first spacer configured to support a portion of a patient's body and having a thickness such that a top surface of the first spacer is substantially flush with a top surface of the energy emitting device when disposed on a platform of a surgical table;
   a second spacer configured to support a second portion of a patient's body and having a top surface that becomes substantially flush with the top surface of the energy emitting device and the top surface of the first spacer when positioned on a platform of a surgical table;
   a bed having a bottom surface that is substantially flush with the top surfaces of each of the energy emitting device, the first spacer, and the second spacer when the energy emitting device, the first spacer, and the second spacer are disposed on a platform of a surgical table and the bed is positioned on the energy emitting device, the first spacer, and the second spacer; and
   a tracking system configured to receive location information from the first and second tracking sensors and to overlay the ultrasound images with a graphical representation of the treatment probe on a display based on the location information.

## Claims

1. A surgical table (ST) including a platform, for supporting a patient (P), and a bed levelling system, the bed leveling system comprising:
an energy emitting device (121) configured and dimensioned to be positioned on said platform of said surgical table, said energy emitting device including at least one fastener (122a-d), configured to detachably couple to said platform; and
a first spacer (200) configured to be positioned on said platform of said surgical table to support a portion of a patient's body and said first spacer includes at least one fastener (201a-e) configured to detachably couple to said platform,
the first spacer having a thickness such that a top surface of the first spacer is substantially flush with a top surface of the energy emitting device when disposed adjacent the energy emitting device on the platform of the surgical table.

2. The surgical table according to claim 1, wherein the first spacer (200) includes an end (200d) which is contoured corresponding to a geometry of an edge (121a) of the energy emitting device (121) such that a substantially continuous surface is formed when the first spacer and the energy emitting device are proximately positioned.

3. The surgical table according to claim 1 or 2, further comprising a second spacer (202) configured to support a second portion of a patient's body and having a top surface that is substantially flush with the top surface of the energy emitting device (121) and the top surface of the first spacer (200) when positioned on the platform of the surgical table.

4. The surgical table according to claim 3, wherein the second spacer has an end having a surface contour corresponding to the geometry of the energy emitting device when positioned adjacent one another.

5. The surgical table according to claim 3 or 4, wherein the second spacer (202) includes at least one fastener (203a-e) configured to detachably couple to said platform.

6. The surgical table according to any preceding claim, wherein the first spacer (200) includes a fastener (201a, 208a 208b)) configured to detachably couple to the energy emitting device.

7. The surgical table according to claim 6, wherein the at least one fastener (208a, 208b) configured to detachably couple to the energy emitting device of the first spacer (200) is pivotable such that the first spacer is movably coupled to the energy emitting device.

8. The surgical table according to any preceding claim, wherein the first spacer (200) includes a moisture barrier (204) .

9. The surgical table according to any preceding claim, wherein the first spacer (200) is constructed from a rigid foam material.

10. The surgical table according to any preceding claim, wherein the first spacer (200) includes a plurality of cells configured to form a grid structure, the grid structure being resistant to a first force in a first direction.

11. The surgical table according to claim 10, wherein the grid structure of the first spacer (200) is configured to be collapsible in response to a second force in a second direction.

12. The surgical table according to any preceding claim, wherein the energy emitting device (121) includes an electromagnetic (EM) field generator.

13. A treatment system comprising:
a treatment probe (130) configured to treat tissue and having a first tracking sensor (137);
an ultrasound imager (140) configured to generate ultrasound images and having a second tracking sensor (141);
an energy emitting device (121);
a tracking system (100) configured to receive location information from the first and second tracking sensors (137, 141) and to overlay the ultrasound images with a graphical representation of the treatment probe on a display based on the location information;
a surgical table according to any preceding claim; and
a bed (120) having a bottom surface that is substantially flush with the top surfaces of each of the energy emitting device (121), the first spacer (200), and the second spacer (202).

## Patentansprüche

1. Operationstisch (ST) mit einer Auflageebene zum Tragen eines Patienten (P) und einem Bettnivelliersystem, wobei das Bettnivelliersystem Folgendes umfasst:
eine Energie abgebende Vorrichtung (121), die dazu ausgebildet und derart dimensioniert ist, um auf der Auflageebene des Operationstischs positioniert zu werden, wobei die Energie abgebende Vorrichtung mindestens ein Befestigungselement (122a-d) aufweist, das dazu ausgebildet ist, abnehmbar mit der Auflageebene verbunden zu werden; und
einen ersten Abstandshalter (200), der dazu ausgebildet ist, auf der Auflageebene des Operationstischs positioniert zu werden, um einen Teil des Körpers des Patienten zu tragen, und wobei der erste Abstandshalter mindestens ein Befestigungselement (201a-e) aufweist, das dazu ausgebildet ist, abnehmbar mit der Auflageebene verbunden zu werden,
wobei der erste Abstandshalter eine derartige Dicke aufweist, dass eine obere Fläche des ersten Abstandshalters im Wesentlichen bündig mit einer oberen Fläche der Energie abgebenden Vorrichtung verläuft, wenn er benachbart zu der Energie abgebenden Vorrichtung auf der Auflageebene des Operationstischs angeordnet wird.

2. Operationstisch nach Anspruch 1, wobei der erste Abstandshalter (200) ein Ende (200d) aufweist, das mit einer Kontur entsprechend einer Geometrie einer Kante (121a) der Energie abgebenden Vorrichtung (121) versehen ist, sodass eine im Wesentlichen durchgehende Oberfläche gebildet wird, wenn der erste Abstandshalter und die Energie abgebende Vorrichtung nahe zueinander positioniert werden.

3. Operationstisch nach Anspruch 1 oder 2, ferner umfassend einen zweiten Abstandshalter (202), der dazu ausgebildet ist, einen zweiten Teil des Körpers des Patienten zu tragen, und eine obere Fläche aufweist, die im Wesentlichen bündig mit der oberen Fläche der Energie abgebenden Vorrichtung (121) und der oberen Fläche des ersten Abstandshalters (200) verläuft, wenn er auf der Auflageebene des Operationstischs positioniert wird.

4. Operationstisch nach Anspruch 3, wobei der zweite Abstandshalter ein Ende mit einer Oberflächenkontur entsprechend der Geometrie der Energie abgebenden Vorrichtung aufweist, wenn sie benachbart zueinander positioniert werden.

5. Operationstisch nach Anspruch 3 oder 4, wobei der zweite Abstandshalter (202) mindestens ein Befestigungselement (203a-e) aufweist, das dazu ausgebildet ist, abnehmbar mit der Auflageebene verbunden zu werden.

6. Operationstisch nach einem vorhergehenden Anspruch, wobei der erste Abstandshalter (200) ein Befestigungselement (201a, 208a 208b) aufweist, das dazu ausgebildet ist, abnehmbar mit der Energie abgebenden Vorrichtung verbunden zu werden.

7. Operationstisch nach Anspruch 6, wobei das mindestens eine Befestigungselement (208a, 208b), das dazu ausgebildet ist, abnehmbar mit der Energie abgebenden Vorrichtung des ersten Abstandshalters (200) verbunden zu werden, derart schwenkbar ist, dass der erste Abstandshalter beweglich mit der Energie abgebenden Vorrichtung verbunden ist.

8. Operationstisch nach einem vorhergehenden Anspruch, wobei der erste Abstandshalter (200) eine Feuchtigkeitsbarriere (204) aufweist.

9. Operationstisch nach einem vorhergehenden Anspruch, wobei der erste Abstandshalter (200) aus einem starren Schaummaterial aufgebaut ist.

10. Operationstisch nach einem vorhergehenden Anspruch, wobei der erste Abstandshalter (200) mehrere Zellen aufweist, die dazu ausgebildet sind, eine Gitterstruktur zu bilden, wobei die Gitterstruktur einer ersten Kraft in einer ersten Richtung widersteht.

11. Operationstisch nach Anspruch 10, wobei die Gitterstruktur des ersten Abstandshalters (200) dazu ausgebildet ist, in Reaktion auf eine zweite Kraft in einer zweiten Richtung zusammendrückbar zu sein.

12. Operationstisch nach einem vorhergehenden Anspruch, wobei die Energie abgebende Vorrichtung (121) einen Generator eines elektromagnetischen (EM) Felds aufweist.

13. Behandlungssystem, umfassend:
eine Behandlungssonde (130), die dazu ausgebildet ist, Gewebe zu behandeln, und einen ersten Ortungssensor (137) aufweist;
einen Ultraschallbildgeber (140), der dazu ausgebildet ist, Ultraschallbilder zu erzeugen, und einen zweiten Ortungssensor (141) aufweist;
eine Energie abgebende Vorrichtung (121);
ein Ortungssystem (100), das dazu ausgebildet ist, Ortsinformationen von dem ersten und dem zweiten Ortungssensor (137, 141) zu empfangen und Ultraschallbilder mit einer graphischen Darstellung der Behandlungssonde auf einer Anzeige auf Basis der Ortsinformationen zu überlagern;
einen Operationstisch nach einem vorhergehenden Anspruch; und
ein Bett (120) mit einer unteren Fläche, die im Wesentlichen bündig mit den oberen Flächen der Energie abgebenden Vorrichtung (121), des ersten Abstandshalters (200) als auch des zweiten Abstandshalters (202) verläuft.

## Revendications

1. Table chirurgicale (ST) incluant une plateforme, pour supporter un patient (P), et un système de mise à niveau de lit, le système de mise à niveau de lit comprenant :
un dispositif d'émission d'énergie (121) configuré et dimensionné pour être positionné sur ladite plateforme de ladite table chirurgicale, ledit dispositif d'émission d'énergie incluant au moins une attache (122a-d), configurée pour se coupler de manière détachable à ladite plateforme ; et
un premier écarteur (200) configuré pour être positionné sur ladite plateforme de ladite table chirurgicale pour supporter une partie du corps d'un patient et ledit premier écarteur inclut au moins une attache (201a-e) configurée pour se coupler de manière détachable à ladite plateforme,
le premier écarteur ayant une épaisseur telle qu'une surface supérieure du premier écarteur est sensiblement à fleur avec une surface supérieure du dispositif d'émission d'énergie quand il est disposé de manière adjacente au dispositif d'émission d'énergie sur la plateforme de la table chirurgicale.

2. Table chirurgicale selon la revendication 1, dans laquelle le premier écarteur (200) inclut une extrémité (200d) qui est profilée pour correspondre à une géométrie d'un bord (121a) du dispositif d'émission d'énergie (121) de telle sorte qu'une surface sensiblement continue est formée quand le premier écarteur et le dispositif d'émission d'énergie sont positionnés à proximité.

3. Table chirurgicale selon la revendication 1 ou 2, comprenant en outre un second écarteur (202) configuré pour supporter une seconde partie du corps d'un patient et ayant une surface supérieure qui est sensiblement à fleur avec la surface supérieure du dispositif d'émission d'énergie (121) et la surface supérieure du premier écarteur (200) quand il est positionné sur la plateforme de la table chirurgicale.

4. Table chirurgicale selon la revendication 3, dans lequel le second écarteur a une extrémité ayant un contour de surface correspondant à la géométrie du dispositif d'émission d'énergie quand ils sont positionnés de manière adjacente l'un à l'autre.

5. Table chirurgicale selon la revendication 3 ou 4, dans lequel le second écarteur (202) inclut au moins une attache (203a-e) configurée pour se coupler de manière détachable à ladite plateforme.

6. Table chirurgicale selon l'une quelconque des revendications précédentes, dans laquelle le premier écarteur (200) inclut une attache (201a, 208a, 208b) configurée pour se coupler de manière détachable au dispositif d'émission d'énergie.

7. Table chirurgicale selon la revendication 6, dans laquelle l'au moins une attache (208a, 208b) configurée pour se coupler de manière détachable au dispositif d'émission d'énergie du premier écarteur (200) peut pivoter de telle sorte que le premier écarteur est couplé de manière mobile au dispositif d'émission d'énergie.

8. Table chirurgicale selon l'une quelconque des revendications précédentes, dans laquelle le premier écarteur (200) inclut une barrière contre l'humidité (204).

9. Table chirurgicale selon l'une quelconque des revendications précédentes, dans laquelle le premier écarteur (200) est construit à partir d'un matériau alvéolaire rigide.

10. Table chirurgicale selon l'une quelconque des revendications précédentes, dans laquelle le premier écarteur (200) inclut une pluralité de cellules configurées pour former une structure de grille, la structure de grille étant résistante à une première force dans une première direction.

11. Table chirurgicale selon la revendication 10, dans laquelle la structure de grille du premier écarteur (200) est configurée pour pouvoir s'affaisser en réponse à une seconde force dans une seconde direction.

12. Table chirurgicale selon l'une quelconque des revendications précédentes, dans laquelle le dispositif d'émission d'énergie (121) inclut un générateur de champ électromagnétique (EM).

13. Système de traitement comprenant :
une sonde de traitement (130) configurée pour traiter du tissu et ayant un premier capteur de suivi (137) ;
un imageur à ultrasons (140) configuré pour générer des images à ultrasons et ayant un second capteur de suivi (141) ;
un dispositif d'émission d'énergie (121) ;
un système de suivi (100) configuré pour recevoir des informations d'emplacement provenant des premier et second capteurs de suivi (137, 141) et pour recouvrir les images à ultrasons d'une représentation graphique de la sonde de traitement sur un dispositif d'affichage sur la base des informations d'emplacement ;
une table chirurgicale selon l'une quelconque des revendications précédentes ; et
un lit (120) ayant une surface de fond qui est sensiblement à fleur avec les surfaces supérieures de chacun du dispositif d'émission d'énergie (121), du premier écarteur (200) et du second écarteur (202).
